# EUROPÄISCHE PATENTSCHRIFT

(11) **EP 4 069 330 B1**
(45) Veröffentlichungstag und Bekanntmachung des Hinweises auf die Patenterteilung: **24.05.2023**
(21) Anmeldenummer: 21777539.4
(22) Anmeldetag: 17.09.2021
(51) Int. Cl.: A61M 1/36

(54) **AUTOMATISCHES PRIMEN EINER EXTRAKORPORALEN BLUTBEHANDLUNGSVORRICHTUNG UNTER VERWENDUNG EINES PUSH-PULL-VERFAHRENS**
AUTOMATIC PRIMING OF AN EXTRACORPOREAL BLOOD TREATMENT DEVICE USING A PUSH-PULL METHOD
AMORÇAGE AUTOMATIQUE D'UN DISPOSITIF DE TRAITEMENT SANGUIN EXTRACORPOREL À L'AIDE D'UN PROCÉDÉ DE POUSSÉE-TRACTION

(30) Priorität: 28.09.2020 DE 102020125291
(43) Veröffentlichungstag der Anmeldung: 12.10.2022
(73) Patentinhaber: B. Braun Avitum AG, 34212 Melsungen (DE)
(72) Erfinder: WÜRSCHMIDT, Tobias, 34346 Hann. Münden (DE); SCHILLER, Joana Sophie, 48429 Rheine (DE)
(74) Vertreter: Winter, Brandl - Partnerschaft mbB
(86) Internationale Anmeldenummer: PCT/EP2021/075701
(87) Internationale Veröffentlichungsnummer: WO 2022/063706

(56) Entgegenhaltungen:
- DE-A1-102019 101 542
- DE-C1- 10 011 208
- US-A1- 2012 265 117
- US-A1- 2020 061 273

## Beschreibung

Die vorliegende Offenbarung betrifft eine extrakorporale Blutbehandlungsvorrichtung, insbesondere Dialysemaschine, welche zum automatischen Primen derselben vorbereitet bzw. eingerichtet ist. Weiterhin betrifft die vorliegende Offenbarung ein Verfahren zum automatischen Primen einer extrakorporalen Blutbehandlungsvorrichtung.

### Technischer Hintergrund

Aus der Praxis sind extrakorporale (Blut-) Kreisläufe zum Führen von Blut außerhalb eines Patientenkörpers während eines Blutreinigungsverfahrens bekannt. Derartige extrakorporale Kreisläufe werden vor Beginn einer Behandlung mit einer Priminglösung bzw. Primingflüssigkeit befüllt. Dieses Befüllen des extrakorporalen Kreislaufs und eines Dialysators mit Priminglösung (z.B. NaCl) bzw. Primingflüssigkeit (Primen) dient einer Vorbereitung des extrakorporalen Kreislaufs und des Dialysators auf die nachfolgende Behandlung. Insbesondere dient das Primen dazu, im extrakorporalen Kreislauf und im Dialysator vor der Behandlung noch vorhandene Luft durch eine physiologisch verträgliche Flüssigkeit oder Lösung zu verdrängen, um ein Einbringen dieser Luft in das Gefäßsystem des Patienten während und insbesondere zu Beginn der Blutbehandlung zu verhindern.

Neben dem Primen bzw. Primingvorgang wird zur Vorbereitung des extrakorporalen Kreislaufs und des Dialysators für die nachfolgende Behandlung außerdem üblicherweise ein Spülen bzw. Spülvorgang des extrakorporalen Kreislaufs und des Dialysators vorgenommen. Das Spülen bzw. der Spülvorgang wird gewöhnlich gleichzeitig oder (unmittelbar) nachfolgend an das Primen bzw. den Primingvorgang durchgeführt. Das Spülen bzw. der Spülvorgang dient insbesondere dazu, dass Reststoffe/ Pyrogene aus dem extrakorporalen Kreislauf und aus dem Dialysator, insbesondere aus einer Membran desselben, herausgespült werden. Derartige Reststoffe/ Pyrogene können einem Produktions-, Verpackungs- oder Aufrüstprozess entstammen und stellen eine potentielle Gefährdung des Patienten dar.

Wenn der Dialysator und ein den extrakorporalen (Blut-)Kreislauf bildendes Schlauchsystem vorbereitet sind, das heißt wenn das Primen und Spülen derselben durchgeführt wurde, kann der Patient grundsätzlich angelegt werden, und die Priming- bzw. Spülflüssigkeit wird aus dem vollständig gefüllten System abgelassen und auf einer Seite durch das Blut des Patienten ersetzt.

Die vorliegende Offenbarung betrifft insbesondere das Primen, also die Vorbereitung der extrakorporalen Blutbehandlungsvorrichtung. Ein Anlegen/ eine Behandlung eines Patienten ist in anderen Worten bevorzugt nicht Gegenstand der vorliegenden Offenbarung, so dass offenbarungsgemäß bevorzugt kein Kontakt der extrakorporalen Blutbehandlungsvorrichtung mit einem Patienten vorgesehen ist.

### Stand der Technik

Aus dem Stand der Technik ist ein Primen! Priming über einen externen Kochsalzbeutel bekannt. Dabei wird ein Kochsalzbeutel mit einem Schlauch des extrakorporalen Kreislaufs (mit einer Seite des Blutschlauchsystems) verbunden und die Primingflüssigkeit wird in das Schlauchsystem und den Dialysator gefüllt. Damit der Dialysator geeignet mit Flüssigkeit gefüllt wird, muss dieser zwischendurch gedreht werden, um noch vorhandene Luftblasen aus dem System zu entfernen. Die verbrauchte Flüssigkeit wird dabei in einem leeren Beutel aufgefangen.

Ferner ist aus dem Stand der Technik das sogenannte Online-Priming bekannt. Beim Online-Priming wird Dialysierflüssigkeit von der Blutbehandlungsvorrichtung bereitgestellt und über einen Substituatanschluss mit Hilfe einer arteriellen Blutpumpe in das Blutschlauchsystem und den Dialysator gegeben. Auch bei diesem Priming-Verfahren muss der Dialysator gedreht werden, um noch vorhandene Luftblasen aus dem System zu entfernen.

Diese beiden genannten Priming-Verfahren haben den Nachteil, dass grundsätzlich manuell vorzunehmende Schritte von einem Anwender erforderlich sind, beispielsweise beim Drehen des Dialysators. Aufgrund eines hohen Kostendrucks und zum Teil strengen Richtlinien (z.B. des Centers for Disease Control and Preventions in den Vereinigten Staaten) ist grundsätzlich eine automatische Vorbereitung, insbesondere ein automatisches Primen, der extrakorporalen Blutbehandlungsvorrichtung wünschenswert. Dabei sollen bevorzugt kein Kochsalzlösungsbeutel und keine bzw. möglichst wenige Einwegartikel, welche vor der Therapie entfernt und entsorgt werden müssen, zum Einsatz kommen.

Aus dem Stand der Technik ist grundsätzlich auch bekannt, dass zum Primen Dialysierflüssigkeit/ Dialysat (anstelle einer Kochsalzlösung) verwendet wird. Dabei wird die Dialysierflüssigkeit über eine Membran des Dialysators in das Blutschlauchsystem befördert. Wenn dabei die arterielle und venöse Blutschlauchleitung kurzgeschlossen werden, kann von der Dialysierflüssigkeitsseite aus mittels einer geeigneten Pumpenbetätigung und mittels geeigneten Ventilstellungen Dialysierflüssigkeit in den Dialysator und das Blutschlauchsystem gepumpt werden und diese somit befüllt werden. In diesem Zusammenhang wird beispielsweise auf die EP 3 127 564 B1 oder die EP 1 457 218 A1 verwiesen.

Weitere automatische Priming-Verfahren sind beispielsweise aus der US 2012/0265117 A1, der US 9,579,440 B2 oder der EP 2 361 643 B1 bekannt.

Die DE 10 2019 101 542 A1 offenbart eine extrakorporale Blutbehandlungsvorrichtung gemäß dem Oberbegriff des Anspruchs 1, welche eine Luftpumpe zum Durchführen eines Integritätstests der Membran aufweist.

Aus der US 2020/0061273 A1 ist es bekannt, einen Flüssigkeitsspiegel in einer Tropfkammer während des Primings mittels einer Luftpumpe anzupassen.

Der Stand der Technik hat insbesondere den Nachteil, dass keine geeigneten automatischen Priming-Verfahren existieren, welche gleichermaßen für High-Flux-Dialysatoren (große Poren in der Dialysatormembran) und für Low-Flux-Dialysatoren (kleine Poren in der Dialysatormembran) verwendet werden können. Insbesondere hat es sich im Stand der Technik bei Low-Flux-Dialysatoren bisher immer als schwierig herausgestellt, Dialysierflüssigkeit über die Dialysatormembran in das Blutschlauchsystem/ den extrakorporalen Kreislauf zu befördern.

### Kurzbeschreibung der Offenbarung

Aufgabe der vorliegenden Offenbarung ist es vor diesem Hintergrund, ein verbessertes automatisches Primen einer Blutbehandlungsvorrichtung bereitzustellen, welches gleichermaßen für High-Flux-Dialysatoren und für Low-Flux-Dialysatoren verwendbar ist. Insbesondere soll ein automatisches Primingverfahren bereitgestellt werden, welches es ermöglicht, dass auch bei Low-Flux-Dialysatoren Dialysierflüssigkeit in geeigneter Weise über die Dialysatormembran in das Blutschlauchsystem/ den extrakorporalen Kreislauf befördert werden kann. Dabei soll eine Anzahl von erforderlichen Einwegartikeln/ Einmalartikeln reduziert werden bzw. sollen bevorzugt wenige/ keine zusätzlichen Einwegartikel/ Einmalartikel und kein Substitutionsanschluss erforderlich sein.

Diese Aufgabe wird durch eine extrakorporale Blutbehandlungsvorrichtung nach Anspruch 1 und ein Verfahren zum automatischen Primen einer extrakorporalen Blutbehandlungsvorrichtung nach Anspruch 11 gelöst. Vorteilhafte Ausführungsformen und Weiterbildungen sind Gegenstand der Unteransprüche und/oder werden nachfolgend erläutert.

Die Offenbarung betrifft zunächst eine extrakorporale Blutbehandlungsvorrichtung, welche zum automatischen Primen derselben vorbereitet ist, mit: einem extrakorporalen Kreislauf, einem Dialysator, einem Dialysierflüssigkeitskreislauf und einer Steuereinheit, wobei der extrakorporale Kreislauf und der Dialysierflüssigkeitskreislauf über eine in dem Dialysator vorgesehene Membran voneinander getrennt sind, die Steuereinheit eingerichtet ist, das Primen derart zu steuern, dass eine Flüssigkeit, insbesondere Priming-/ Dialysierflüssigkeit, von dem Dialysierflüssigkeitskreislauf über die Membran des Dialysators an den extrakorporalen Kreislauf geliefert wird, und in dem Dialysierflüssigkeitskreislauf vorgesehene Ventile und/oder Pumpen derart anzusteuern, dass ein Druckaufbau in dem Dialysator auf der Dialysierflüssigkeitsseite erfolgt, wobei die Steuereinheit eine in dem extrakorporalen Kreislauf vorgesehene Strömungsmaschine, insbesondere Kompressor oder Pumpe, ansteuert, insbesondere zyklisch alternierend, einen Push-/ bzw. Druck-Zyklus, in welchem die Strömungsmaschine Luft in den extrakorporalen Kreislauf drückt, und einen Pull-/ bzw. Zug-Zyklus, in welchem die Strömungsmaschine Luft aus dem extrakorporalen Kreislauf herauszieht, auszuführen, zum Unterstützen eines Übertritts der Flüssigkeit über die Membran des Dialysators beim Primen.

Offenbarungsgemäß wird somit grundsätzlich ein automatisches Primen (durch eine geeignete Steuerung) bereitgestellt, bei welchem auf der Dialysierflüssigkeitsseite/ in dem Dialysierflüssigkeitskreislauf ein Druck in dem Dialysator aufgebaut wird. Dieser Druckaufbau kann grundsätzlich in beliebiger Weise durch Ansteuern von in dem Dialysierflüssigkeitskreislauf vorgesehenen Pumpen (beispielsweise Flusspumpe-Eingang und Flusspumpe-Ausgang) bzw. Ventilen (beispielsweise Dialysatoreinlassventil und Dialysatorauslassventil) erfolgen. Dabei wird der Druckaufbau im Wesentlichen dadurch realisiert, dass mehr Flüssigkeit, insbesondere Dialysierflüssigkeit, in den Dialysator gedrückt wird, als aus dem Dialysator abfließen kann. Dabei ist es grundsätzlich auch denkbar, dass die Flüssigkeit etwa durch Schließen des Ventils am Dialysatorausgang (Dialysatorauslassventil) überhaupt nicht mehr aus dem Dialysator abfließen kann. Durch den Druckaufbau in dem Dialysator auf der Dialysierflüssigkeitsseite entsteht ein großer negativer Transmembrandruck (TMP).

Die vorliegende Offenbarung ermöglicht es insbesondere, dass ein Flüssigkeitsübertritt von der Seite des Dialysierflüssigkeitskreislaufs über die Membran des Dialysators zu dem extrakorporalen Kreislauf unterstützt wird, und gleichzeitig der maximal zulässige Transmembrandruck betragsmäßig nicht überschritten wird (das heißt der (negative) vorbestimmte Schwellwert nicht unterschritten wird). Der maximale Transmembrandruck ist vom Dialysatortyp/ vom verwendeten Dialysator abhängig, und ist grundsätzlich in Datenblättern von Dialysatoren mit angegeben.

Insbesondere wird offenbarungsgemäß eine Push-Pull-Steuerung bzw. ein Push-Pull-Verfahren bereitgestellt, welche bzw. welches durch eine geeignete Ansteuerung einer in dem extrakorporalen Kreislauf (der Blutseite) vorgesehenen Strömungsmaschine realisiert wird. Dieses Push-Pull-Verfahren ist bevorzugt in zwei Zyklen unterteilt, nämlich den Druck-/ bzw. Push-Zyklus und den Zug-/ bzw. Pull-Zyklus.

In dem Zug-/ bzw. Pull-Zyklus wird grundsätzlich Luft aus dem extrakorporalen Kreislauf herausgezogen, so dass ein Übertritt von Dialysierflüssigkeit durch die Membran des Dialysators unterstützt und somit erleichtert wird. Jedoch nimmt der Transmembrandruck in nachteilhafter Weise betragsmäßig zu, so dass zum einen die Gefahr besteht, dass der maximal zulässige Transmembrandruck (betragsmäßig) überschritten wird, und zum anderen die Gefahr besteht, dass eine in dem extrakorporalen Kreislauf vorgesehene Luftfalle aufgrund des erzeugten Unterdrucks (negativen Drucks) kollabiert.

Daher ist es offenbarungsgemäß erforderlich, dass der Zug-/ bzw. Pull-Zyklus nicht zu lange ausgeführt wird, und dass auf den Zug-/ bzw. Pull-Zyklus ein Druck-/ bzw. Push-Zyklus folgt. Beim Druck-/ bzw. Push-Zyklus wird bevorzugt (durch die in dem extrakorporalen Kreislauf vorgesehene Strömungsmaschine) Luft in das Schlauchsystem/ den extrakorporalen Kreislauf gedrückt, damit der in dem extrakorporalen Kreislauf vorherrschende Unterdruck neutralisiert wird. Es erfolgt somit in dem Druck-/ bzw. Push-Zyklus lediglich eine Neutralisierung des Unterdrucks, das heißt es wird kein Überdruck erzeugt bzw. aufgebaut.

In anderen Worten beeinflusst der Druck-/ bzw. Push-Zyklus den Transmembrandruck positiv, das heißt der Transmembrandruck wird größer bzw. betragsmäßig kleiner (negativer Druck), und beeinflusst der Zug-/ bzw. Pull-Zyklus den Transmembrandruck negativ, das heißt der Transmembrandruck wird kleiner bzw. betragsmäßig größer (negativer Druck). Durch die offenbarungsgemäße Ansteuerung der Strömungsmaschine auf der Seite des extrakorporalen Kreislaufs wird erreicht, dass der Flüssigkeitsübertritt über die Membran des Dialysators in geeigneter Weise unterstützt wird, und gleichzeitig der maximal zulässige Transmembrandruck betragsmäßig nicht überschritten wird, sowie keine in dem extrakorporalen Kreislauf vorgesehene Luftfalle kollabiert.

Durch die vorliegende Offenbarung wird insgesamt ein automatisches Primen mit Low-Flux-Dialysatoren ermöglicht. Insbesondere wird durch das offenbarungsgemäße Push-Pull-Verfahren bzw. die offenbarungsgemäße Push-Pull-Steuerung erreicht, dass auch bei Low-Flux-Dialysatoren (kleine Poren) ein geeigneter Flüssigkeitsübertritt durch die Membran des Dialysators bewirkt wird. Die vorliegende Offenbarung ist jedoch nicht auf Low-Flux-Dialysatoren beschränkt. Insbesondere gilt, dass das offenbarungsgemäße Verfahren bzw. die offenbarungsgemäße Steuerung auch bei High-Flux-Dialysatoren angewandt werden kann. Grundsätzlich wird gemäß der vorliegenden Offenbarung unter einem Low-Flux-Dialysator ein Dialysator verstanden, welcher einen Ultrafiltrationskoeffizienten KUF von kleiner oder gleich 15 ml/(h*mmHg) (stündliche Ultrafiltration in ml, die pro mmHg Transmembrandruck (TMP) erzielt wird) hat, und wird unter einem High-Flux-Dialysator ein Dialysator verstanden, welcher einen Ultrafiltrationskoeffizienten KUF von größer 15 ml/(h*mmHg) hat.

Es ist von Vorteil, wenn in dem Dialysierflüssigkeitskreislauf stromaufwärts des Dialysators eine Flusspumpe-Eingang und stromabwärts des Dialysators eine Flusspumpe-Ausgang vorgesehen sind, und die Steuereinheit eingerichtet ist, zum Druckaufbau in dem Dialysator auf der Dialysierflüssigkeitsseite die Flusspumpe-Eingang und die Flusspumpe-Ausgang derart anzusteuern, dass eine Flussrate der Flusspumpe-Eingang größer ist als eine Flussrate der Flusspumpe-Ausgang. Somit wird erreicht, dass mehr Flüssigkeit von der Flusspumpe-Eingang in den Dialysator gedrückt wird als von der Flusspumpe-Ausgang entzogen wird, so dass ein geeigneter Druckaufbau in dem Dialysator erfolgen kann.

Bevorzugt werden dabei die Flusspumpe-Eingang und die Flusspumpe-Ausgang mit einer Flussratendifferenz von 300 ml/min bis 500 ml/min, besondere bevorzugt von etwa/ näherungsweise 400 ml/min betrieben. Das bedeutet, dass bevorzugt die Flussrate der Flusspumpe-Eingang um 300 ml/min bis 500 ml/min, besondere bevorzugt um etwa 400 ml/min größer ist als die Flussrate der Flusspumpe-Ausgang.

Bevorzugt ist die in dem extrakorporalen Kreislauf vorgesehene Strömungsmaschine ein Kompressor bzw. eine Levelregulierungspumpe, welcher bzw. welche hinter einer in dem extrakorporalen Kreislauf vorgesehenen Expansionskammer bzw. Luftfalle (beispielsweise einer venösen Luftfalle oder einer arteriellen Luftfalle) angeordnet ist bzw. mit der Luftfalle in Verbindung steht, und die Luft in die Luftfalle drückt oder aus dieser herauszieht. Ein solcher Kompressor bzw. eine solche Pegel-/ Levelregulierungspumpe ist häufig in extrakorporalen Blutbehandlungsvorrichtungen bzw. Dialysemaschinen standardmäßig verbaut bzw. integriert und dient zumeist einer Pegeleinstellung der Luftfalle. Wenn nun dieser Kompressor bzw. diese Levelregulierungspumpe für die Push-Pull-Steuerung der vorliegenden Offenbarung verwendet wird, müssen an der Dialysemaschine nach aktuellem Stand der Technik bevorzugt keine hardwaretechnischen Änderungen, sondern lediglich softwaretechnische Änderungen vorgenommen werden.

In vorteilhafter Weise ist die Steuereinheit eingerichtet, den Push-/ bzw. Druck-Zyklus und den Pull-/ bzw. Zug-Zyklus zyklisch abzuwechseln/ zu alternieren/ zu wiederholen. In anderen Worten werden der Push-/ bzw. Druck-Zyklus und der Pull-/ bzw. Zug-Zyklus mehrfach und wiederholt ausgeführt. Beispielsweise kann zunächst der Push-Zyklus, dann der Pull-Zyklus, dann wieder der Push-Zyklus, anschließend der Pull-Zyklus, usw. ausgeführt werden. Es ist jedoch auch denkbar, dass zunächst der Pull-Zyklus, dann der Push-Zyklus, dann wieder der Pull-Zyklus, usw. ausgeführt werden. In anderen Worten zeichnet sich das offenbarungsgemäße Push-Pull-Verfahren bzw. die offenbarungsgemäße Push-Pull-Steuerung insbesondere durch das zyklische Wiederholen/ Abwechseln/ Alternieren des Push-Zyklus und des Pull-Zyklus aus.

Bevorzugt ist die Steuereinheit eingerichtet, das zyklische Alternieren des Push-/ bzw. Druck-Zyklus und des Pull-/ bzw. Zug-Zyklus abzubrechen, wenn ein in dem venösen Abschnitt des extrakorporalen Kreislaufs vorgesehener Sicherheitsluftdetektor die Flüssigkeit, insbesondere Dialysierflüssigkeit, erfasst. In anderen Worten werden offenbarungsgemäß die Push- und Pull-Zyklen so lange abgewechselt und wiederholt, bis das hier genannte Abbruchkriterium erfüllt ist.

In vorteilhafter Weise ist der Dialysator ein Low-Flux-Dialysator mit einem Ultrafiltrationskoeffizienten von kleiner oder gleich 15 ml/(h*mmHg). Insbesondere hat sich offenbarungsgemäß herausgestellt, dass es mit der beschriebenen Push-Pull-Steuerung möglich ist, nicht nur einen High-Flux-Dialysator, sondern auch über einen Low-Flux-Dialysator in geeigneter Weise automatisch zu primen.

Bevorzugt ist die Steuereinheit eingerichtet, einen Wechsel zwischen dem Push-/ bzw. Druck-Zyklus und dem Pull-/ bzw. Zug-Zyklus sensorgesteuert und/oder zeitgesteuert vorzunehmen. Gemäß der vorliegenden Offenbarung kann somit der Wechsel zwischen Push-Zyklus und Pull-Zyklus und umgekehrt vorgenommen werden, wenn eine vorbestimmte Zeitspanne verstrichen ist. Es ist jedoch auch denkbar, dass über in dem extrakorporalen Kreislauf und/oder dem Dialysierflüssigkeitskreislauf vorgesehene (Druck-) Sensoren Drücke in dem extrakorporalen Kreislauf und/oder dem Dialysierflüssigkeitskreislauf gemessen werden, und der Wechsel zwischen Push-Zyklus und Pull-Zyklus auf der Grundlage der von dem Sensor/ den Sensoren erhaltenen Informationen erfolgt. Beispielsweise kann aus den gemessenen Drücken ein Transmembrandruck berechnet werden, und ein sensorgesteuerter Wechsel zwischen Push-Zyklus und Pull-Zyklus kann auf der Grundlage des berechneten Transmembrandrucks vorgenommen werden. Grundsätzlich ist es offenbarungsgemäß jedoch auch denkbar, dass sowohl Informationen von Sensoren als auch zeitliche Aspekte beim Wechsel zwischen Push-Zyklus und Pull-Zyklus berücksichtigt werden, so dass der Wechsel grundsätzlich auch sensorgesteuert und zeitgesteuert vorgenommen werden kann.

Gemäß einem vorteilhaften Aspekt der vorliegenden Offenbarung ist die Steuereinheit eingerichtet, zuerst den Push-/ bzw. Druck-Zyklus durchzuführen und nach dem erstmaligen Durchführen des Push-/ bzw. Druck-Zyklus den Pull-/ bzw. Zug-Zyklus durchzuführen. Insbesondere hat sich offenbarungsgemäß herausgestellt, dass, wenn zu Beginn des Verfahrens/ der Steuerung der Druck-Zyklus ausgeführt wird, die Flüssigkeit, insbesondere Dialysierflüssigkeit, in dem extrakorporalen Kreislauf nicht unkontrolliert zu einem Drucksensor/ zu der Strömungsmaschine/ zu einem möglicherweise vorgesehenen Hydrophobfilter (vor dem Drucksensor oder der Strömungsmaschine) gelangt.

Ein vorteilhaftes Ausführungsbeispiel der vorliegenden Offenbarung ist dadurch gekennzeichnet, dass die Steuereinheit eingerichtet ist, von dem Push-/ bzw. Druck-Zyklus zu dem Pull-/ bzw. Zug-Zyklus zu wechseln, wenn ein Transmembrandruck einen ersten vorbestimmten Schwellwert überschreitet, und von dem Pull-/ bzw. Zug-Zyklus zu dem Push-/ bzw. Druck-Zyklus zu wechseln, wenn der Transmembrandruck einen zweiten vorbestimmten Schwellwert unterschreitet.

Grundsätzlich gilt, dass beim Push-Zyklus der Transmembrandruck größer wird bzw. betragsmäßig abnimmt (negativer Wert). Wenn der Transmembrandruck größer als der erste vorbestimmte Schwellwert ist bzw. wird, erfolgt der Wechsel zu dem Pull-Zyklus. Offenbarungsgemäß hat sich herausgestellt, dass der erste vorbestimmte Schwellwert bevorzugt bei näherungsweise/ gerundet/ etwa -400 mmHg eingestellt werden sollte. Beim Pull-Zyklus gilt, dass der Transmembrandruck kleiner wird bzw. betragsmäßig zunimmt (negativer Wert). Wenn der Transmembrandruck dabei kleiner als der zweite vorbestimmte Schwellwert ist bzw. wird, erfolgt der Wechsel zu dem Push-Zyklus. Offenbarungsgemäß hat sich herausgestellt, dass der zweite vorbestimmte Schwellwert bevorzugt bei näherungsweise/ gerundet/ etwa -420 mmHg eingestellt werden sollte.

Es gilt somit, dass bevorzugt der erste vorbestimmte Schwellwert größer als der zweite vorbestimmte Schwellwert ist.

Bevorzugt ist die Steuereinheit eingerichtet, den Wechsel zwischen dem Push-/ bzw. Druck-Zyklus und dem Pull-/ bzw. Zug-Zyklus in Abhängigkeit von dem verwendeten Dialysator vorzunehmen. In anderen Worten kann offenbarungsgemäß vorgesehen sein, dass das Push-Pull-Verfahren bzw. die Push-Pull-Steuerung in Abhängigkeit des verwendeten Dialysators (High-Flux-Dialysator oder Low-Flux-Dialysator) durchgeführt wird, also dass sich die Steuerung bei einem High-Flux-Dialysator von der Steuerung bei einem Low-Flux-Dialysator unterscheidet.

Weiterhin betrifft die vorliegende Offenbarung ein Verfahren zum automatischen Primen einer extrakorporalen Blutbehandlungsvorrichtung umfassend einen extrakorporalen Kreislauf, einen Dialysator und einen Dialysierflüssigkeitskreislauf mit den Schritten: Primen des extrakorporalen Kreislaufs und des Dialysators, indem eine Flüssigkeit von dem Dialysierflüssigkeitskreislauf über eine Membran des Dialysators an den extrakorporalen Kreislauf geliefert wird; Aufbauen eines Drucks in dem Dialysator auf der Dialysierflüssigkeitsseite durch geeignetes Ansteuern bzw. Betätigen von in dem Dialysierflüssigkeitskreislauf vorgesehenen Ventilen und/oder Pumpen; und Ausführen eines Push-/ bzw. Druck-Zyklus, in welchem eine in dem extrakorporalen Kreislauf vorgesehene Strömungsmaschine, insbesondere Kompressor oder Pumpe, Luft in den extrakorporalen Kreislauf drückt, und eines Pull-/ bzw. Zug-Zyklus, in welchem die Strömungsmaschine Luft aus dem extrakorporalen Kreislauf herauszieht, zum Unterstützen eines Übertritts der Flüssigkeit über die Membran des Dialysators beim Primen.

Bevorzugt weist das Verfahren weiterhin den folgenden Schritt auf: Betreiben einer Flusspumpe-Eingang stromaufwärts des Dialysators mit einer Flussrate, die größer ist als eine Flussrate einer Flusspumpe-Ausgang stromabwärts des Dialysators.

In vorteilhafter Weise weist das Verfahren ferner den folgenden Schritt auf: Drücken der Luft in eine und Herausziehen der Luft aus einer in dem extrakorporalen Kreislauf vorgesehenen Expansionskammer durch einen Kompressor bzw. eine Pegel-/ Levelregulierungspumpe.

Weiterhin ist es von Vorteil, wenn das Verfahren folgenden Schritt aufweist: Zyklisches Abwechseln und Wiederholen des Push-/ bzw. Druck-Zyklus und des Pull-/ bzw. Zug-Zyklus.

Bevorzugt weist das Verfahren weiterhin den folgenden Schritt auf: Abbrechen des zyklischen Alternierens des Push-/ bzw. Druck-Zyklus und des Pull-/ bzw. Zug-Zyklus, wenn ein in einem venösen Abschnitt des extrakorporalen Kreislaufs vorgesehener Sicherheitsluftdetektor die Flüssigkeit, insbesondere Dialysierflüssigkeit, erfasst.

Das Verfahren weist bevorzugt ferner den folgenden Schritt auf: Zeitgesteuertes und/oder sensorgesteuertes Wechseln zwischen dem Push-/ bzw. Druck-Zyklus und dem Pull-/ bzw. Zug-Zyklus.

Bevorzugt wird gemäß dem offenbarungsgemäßen Verfahren zuerst der Push-/ bzw. Druck-Zyklus durchgeführt und nach dem erstmaligen Durchführen des Push-/ bzw. Druck-Zyklus der Pull-/ bzw. Zug-Zyklus durchgeführt.

In vorteilhafter Weise weist das Verfahren weiterhin den folgenden Schritt auf: Wechseln von dem Push-/ bzw. Druck-Zyklus zu dem Pull-/ bzw. Zug-Zyklus, wenn ein Transmembrandruck einen ersten vorbestimmten Schwellwert überschreitet, und Wechseln von dem Pull-/ bzw. Zug-Zyklus zu dem Push-/ bzw. Druck-Zyklus, wenn der Transmembrandruck einen zweiten vorbestimmten Schwellwert unterschreitet.

### Kurzbeschreibung der Figuren

Die Offenbarung wird nachfolgend anhand von Figuren weiter erläutert. Es zeigen:
- Fig. 1: eine extrakorporale Blutbehandlungsvorrichtung gemäß einem ersten bevorzugten Ausführungsbeispiel der vorliegenden Offenbarung;
- Fig. 2: eine extrakorporale Blutbehandlungsvorrichtung gemäß einem zweiten bevorzugten Ausführungsbeispiel der vorliegenden Offenbarung;
- Fig. 3: ein Flussdiagramm eines automatischen Primingverfahrens gemäß der vorliegenden Offenbarung;
- Fig. 4: einen Ablaufplan des offenbarungsgemäßen Push-Pull-Verfahrens; und
- Fig. 5: ein Diagramm, in welchem der Verlauf des Transmembrandrucks beim offenbarungsgemäßen Push-Pull-Verfahren veranschaulicht ist.

### Figurenbeschreibung

Die Figuren sind lediglich schematischer Natur und dienen ausschließlich dem Verständnis der vorliegenden Offenbarung. Gleiche Elemente sind dabei mit den gleichen Bezugszeichen bezeichnet. Die Merkmale der einzelnen Ausführungsbeispiele können untereinander ausgetauscht werden.

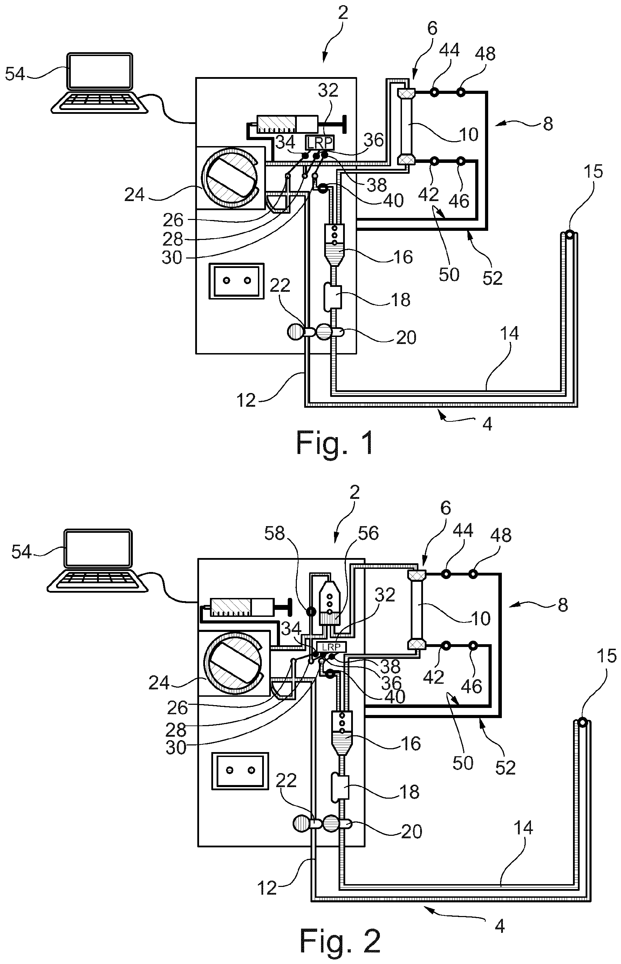

Fig. 1 zeigt eine extrakorporale Blutbehandlungsvorrichtung (Dialysemaschine) 2 gemäß einem ersten bevorzugten Ausführungsbeispiel der vorliegenden Offenbarung.

Die extrakorporale Blutbehandlungsvorrichtung 2 enthält grundsätzlich einen extrakorporalen Kreislauf (A/V-Schlauchsystem) 4, einen Dialysator 6 und einen Dialysierflüssigkeitskreislauf 8. Dabei sind der extrakorporale Kreislauf 4 und der Dialysierflüssigkeitskreislauf 8 über eine in dem Dialysator 6 vorgesehene Membran 10 voneinander getrennt.

Der extrakorporale Kreislauf 4 enthält einen arteriellen Abschnitt 12, welcher sich stromaufwärts des Dialysators 6 befindet, und einen venösen Abschnitt 14, welcher sich stromabwärts des Dialysators 6 befindet.

Wie aus Fig. 1 ersichtlich ist, sind der arterielle Abschnitt 12 und der venöse Abschnitt 14 über einen Adapter bzw. Verbinder 15 verbunden/ kurzgeschlossen. In anderen Worten sind sowohl ein Ende des arteriellen Abschnitts 12 als auch ein Ende des venösen Abschnitts 14 in den Adapter 15 eingesteckt, damit der arterielle Abschnitt 12 und der venöse Abschnitt 14 fluidisch miteinander verbunden sind. Bevorzugt können in den Adapter 15 zwei Luer-Anschlüsse, welche bevorzugt an den Enden des arteriellen Abschnitts 12 und des venösen Abschnitts 14 vorgesehen sind, eingesteckt werden.

In dem venösen Abschnitt 14 des extrakorporalen Kreislaufs 4 sind stromabwärts des Dialysators 6 (das heißt ausgehend von dem Dialysator 6 in einer Richtung hin zu dem Ende des venösen Abschnitts 14) eine venöse Expansionskammer bzw. Luftfalle 16, ein venöser Sicherheitsluftdetektor 18 und eine venöse Schlauchklemme 20 vorgesehen.

In dem arteriellen Abschnitt 12 sind ausgehend von dem Adapter 15 in einer Richtung hin zu dem Dialysator 6 eine arterielle Schlauchklemme 22 und eine (arterielle) Blutpumpe 24 vorgesehen. Wie in Fig. 1 ersichtlich ist, ist der extrakorporale Kreislauf 4 (insbesondere ein Blutpumpenadapter desselben) bereits in die Blutpumpe 24, welche bevorzugt als Rollenpumpe bzw. Peristaltikpumpe ausgebildet ist und eingerichtet ist, durch Quetschen eines Schlauchs ein Fluid/ eine Flüssigkeit zu fördern, eingesetzt.

In dem arteriellen Abschnitt 12 kann ein arterieller Druck stromaufwärts bzw. vor der Blutpumpe 24 mit einem arteriellen Drucksensor 26 gemessen werden. Weiterhin kann ein Dialysatoreingangsdruck stromabwärts bzw. nach der Blutpumpe 24 und stromaufwärts bzw. vor dem Dialysator 6 (zwischen Dialysator 6 und Blutpumpe 24) über einen Dialysatoreingangsdrucksensor 28 gemessen werden. In dem venösen Abschnitt 14 kann ein venöser Druck an/ hinter der venösen Expansionskammer bzw. Luftfalle 16 über einen venösen Drucksensor 30 gemessen werden. Die in dem extrakorporalen Kreislauf 4 vorgesehenen Drucksensoren 26, 28, 30 können den Druck an den entsprechenden Stellen in dem extrakorporalen Kreislauf 4, an welchen diese angeordnet/ vorgesehen sind, messen/ abnehmen/ überwachen.

Wie aus Fig. 1 weiterhin hervorgeht, befindet sich hinter dem arteriellen Drucksensor 26, dem Dialysatoreingangsdrucksensor 28 und dem venösen Drucksensor 30 ein Kompressor bzw. eine Pegel- oder Levelregulierungspumpe (LRP) 32 (als ein Beispiel einer Strömungsmaschine gemäß der vorliegenden Offenbarung) mit dazugehörigen Ventilen 34, 36, 38, nämlich einem ersten Ventil 34 zwischen dem arteriellen Drucksensor 26 und der Pegel- oder Levelregulierungspumpe 32, einem zweiten Ventil 36 zwischen dem Dialysatoreingangsdrucksensor 28 und der Pegel- oder Levelregulierungspumpe 32, und einem dritten Ventil 38 zwischen dem venösen Drucksensor 30 und der Pegel- oder Levelregulierungspumpe 32.

Zwischen der venösen Expansionskammer 16 und dem venösen Drucksensor 30 ist ein Hydrophobfilter 40 vorgesehen, welcher es ermöglicht, dass Flüssigkeit von dem venösen Drucksensor 30 und der Pegel- oder Levelregulierungspumpe 32 ferngehalten wird.

Der Dialysierflüssigkeitskreislauf 8 enthält ein Dialysatoreinlassventil 42, ein Dialysatorauslassventil 44, eine Flusspumpe-Eingang 46 und eine Flusspumpe-Ausgang 48. Das Dialysatoreinlassventil 42 und die Flusspumpe-Eingang 46 sind dabei an einem Dialysierflüssigkeitszufluss 50 stromaufwärts des Dialysators 6 vorgesehen/ angeordnet. Das Dialysatorauslassventil 44 und die Flusspumpe-Ausgang 48 sind an einem Dialysierflüssigkeitsabfluss 52 stromabwärts des Dialysators 6 vorgesehen/ angeordnet. Die Flusspumpe-Eingang 46 und die Flusspumpe-Ausgang 48 sind bevorzugt Zahnradpumpen.

Die extrakorporale Blutbehandlungsvorrichtung 2 weist weiterhin eine Steuereinheit 54 auf, welche bevorzugt als ein Prozessor, insbesondere als eine zentrale Rechen-/ bzw.

Verarbeitungseinheit (CPU), ausgebildet ist. Die Steuereinheit 54 erhält Informationen von Sensoren, welche in der extrakorporalen Blutbehandlungsvorrichtung 2 vorgesehen sind. Lediglich beispielhaft sind dabei die in Fig. 1 gezeigten Sensoren zu nennen, also der arterielle Drucksensor 26, der Dialysatoreingangsdrucksensor 28, der venöse Drucksensor 30, der venöse Sicherheitsluftdetektor 18, etc.. Auf der anderen Seite steuert bzw. betätigt die Steuereinheit 54 Aktoren, welche in der extrakorporalen Blutbehandlungsvorrichtung 2 vorgesehen sind. Lediglich beispielhaft sind dabei die in Fig. 1 gezeigten Ventile oder Pumpen, also insbesondere das Dialysatoreinlassventil 42, das Dialysatorauslassventil 44, die Flusspumpe-Eingang 46, die Flusspumpe-Ausgang 48, die (arterielle) Blutpumpe 24, die Ventile 34, 36, 38 etc. zu nennen.

Fig. 2 zeigt eine extrakorporale Blutbehandlungsvorrichtung (Dialysemaschine) 2 gemäß einem zweiten bevorzugten Ausführungsbeispiel der vorliegenden Offenbarung. Wie aus Fig. 2 hervorgeht, ist das zweite bevorzugte Ausführungsbeispiel dem ersten bevorzugten Ausführungsbeispiel sehr ähnlich, so dass die voranstehenden Ausführungen bzw. Beschreibungen mutatis mutandis für den in Fig. 2 gezeigten Aufbau gelten und daher nicht wiederholt werden. Vor diesem Hintergrund werden in den nachfolgenden Ausführungen lediglich die Unterschiede der zweiten Ausführungsform im Vergleich zur ersten Ausführungsform erläutert.

Wie aus Fig. 2 hervorgeht, weist der extrakorporale Kreislauf 4 neben der venösen Expansionskammer bzw. Luftfalle 16 auch eine arterielle Expansionskammer bzw.

Luftfalle 56 auf. Die arterielle Expansionskammer 56 ist mit dem Dialysatoreingangsdrucksensor 28 verbunden bzw. an diesen angeschlossen.

Der Kompressor bzw. die Pegel-/ Levelregulierungspumpe 32 kann gemäß diesem Ausführungsbeispiel Luft sowohl in die arterielle Expansionskammer 56 als auch in die venösen Expansionskammer 16 hineindrücken bzw. aus diesen herausziehen, das heißt der Kompressor bzw. die Pegel-/ Levelregulierungspumpe 32 kann seine/ihre offenbarungsgemäß beabsichtigte Funktion sowohl an der arteriellen Expansionskammer 56 als auch an der venösen Expansionskammer 16 ausüben. Die Pegel-/ Levelregulierungspumpe 32 kann, wie aus Fig. 2 hervorgeht, jedoch an den beiden Expansionskammern 16, 56 nicht entgegengesetzt arbeiten, das heißt keine entgegengesetzten Funktionen ausüben. Das heißt, die Pegel-/ Levelregulierungspumpe 32 kann nicht an der einen Luftfalle 16 oder 56 Luft in den extrakorporalen Kreislauf 4 drücken und an der anderen Luftfalle 56 oder 16 Luft aus dem extrakorporalen Kreislauf 4 entfernen. Eine separate Ansteuerung der Luftfallen 16, 56 ist jedoch möglich, indem entweder die venöse Luftfalle 16 durch das dritte Ventil 38 von der Pegel-/ Levelregulierungspumpe 32 abgetrennt wird (zur Ansteuerung der arteriellen Luftfalle 56) oder die arterielle Luftfalle 56 durch das zweite Ventil 36 von der Pegel-/ Levelregulierungspumpe 32 abgetrennt wird (zur Ansteuerung der venösen Luftfalle 16).

Wie weiterhin aus Fig. 2 hervorgeht, ist auch zwischen der arteriellen Luftfalle 56 und dem Dialysatoreingangsdrucksensor 28 ein Hydrophobfilter 58 vorgesehen, welcher es ermöglicht, dass Flüssigkeit von dem Dialysatoreingangsdrucksensor 28 und der Pegel- oder Levelregulierungspumpe 32 ferngehalten wird.

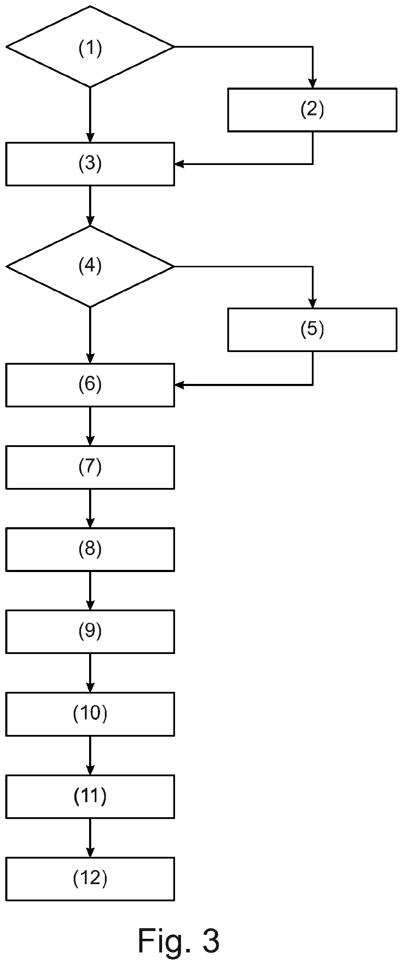

Fig. 3 zeigt ein Flussdiagramm eines Verfahrensablaufs des automatischen Primens gemäß der vorliegenden Offenbarung. Die Verfahrensabläufe der beiden in Fig. 1 und Fig. 2 gezeigten Ausführungsbeispiele sind grundsätzlich sehr ähnlich, so dass in den nachfolgenden Ausführungen lediglich auf Unterschiede zwischen den jeweiligen Verfahrens-/ bzw. Programmabläufen eingegangen werden wird.

Zu Beginn des Verfahrens wird bei (1) "Membranbewegung?" überprüft, ob sich eine Membran in einer Bilanzkammer (nicht gezeigt) in der extrakorporalen Blutbehandlungsvorrichtung 2 bewegt. Ist dies der Fall (JA), wird die Membran in der Bilanzkammer bei (2) "Membran stellen" gestellt. Dabei wird die Bilanzkammer auf Durchfluss gestellt, wodurch ermöglicht wird, dass die Flusspumpe-Eingang 46 und die Flusspumpe-Ausgang 48 unterschiedliche Flüsse bzw. Flussraten erzeugen können. Wird keine Membranbewegung festgestellt (NEIN) bzw. ist die Membran gestellt worden, geht das Verfahren von (1) oder (2) zu (3) "Dialysator dialysierflüssigkeitsseitig befüllen" über.

Bei (3) wird der Dialysator 6 auf der Dialysierflüssigkeitsseite mit (Dialysier-)Flüssigkeit gefüllt. Dazu werden die in dem Dialysierflüssigkeitskreislauf 8 vorgesehenen Ventile und Pumpen in geeigneter Weise angesteuert. Bevorzugt werden die Flusspumpe-Eingang 46 und die Flusspumpe-Ausgang 48 mit einer (geringen) Flussratendifferenz betrieben. Beispielsweise kann die Flussrate der Flusspumpe-Eingang 46 auf 500 ml/min und die Flussrate der Flusspumpe-Ausgang 48 auf 470 ml/min eingestellt werden. Bevorzugt sind in diesem Schritt die arterielle Schlauchklemme 22 und die venöse Schlauchklemme 20 geöffnet und die arterielle Blutpumpe 24 dreht sich nicht. Schritt (3) wird bevorzugt zeitgesteuert abgebrochen. Das heißt, wenn eine vorbestimmte Zeitdauer verstrichen ist (beispielsweise 40 Sekunden), wird angenommen, dass der Dialysator 6 in geeigneter Weise mit (Dialysier-)Flüssigkeit befüllt wurde.

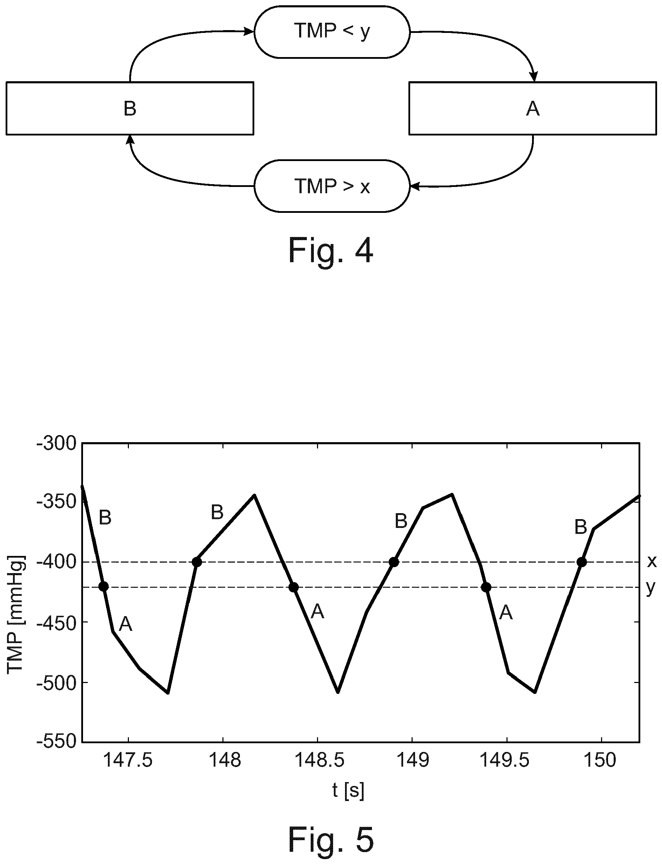

Bei (4) "Push/Pull universal" in Fig. 3 wird schließlich das den Kernaspekt der vorliegenden Offenbarung bildende Push-Pull-Verfahren ausgeführt. Wie unter Bezugnahme auf Fig. 4 deutlich wird, unterteilt sich das offenbarungsgemäße Push-Pull-Verfahren in zwei Zyklen, nämlich den Push-/ bzw. Druck-Zyklus A und den Pull-/ bzw. Zug-Zyklus B, die zyklisch wiederholt/ abgewechselt werden.

Beim Push-Pull-Verfahren soll zum Primen grundsätzlich eine (Dialysier-)Flüssigkeit von dem Dialysierflüssigkeitskreislauf 8 über die Membran 10 des Dialysators 6 an den extrakorporalen Kreislauf 4 geliefert werden. Dabei wird zunächst auf der Dialysierflüssigkeitsseite/ in dem Dialysierflüssigkeitskreislauf 8 ein Druck in dem Dialysator 6 aufgebaut. Dies erfolgt offenbarungsgemäß bevorzugt dadurch, dass die Flusspumpe-Eingang 46 und die Flusspumpe-Ausgang 48 von der Steuereinheit 54 derart angesteuert werden, dass eine Flussrate der Flusspumpe-Eingang 46 (wesentlich) größer ist als eine Flussrate der Flusspumpe-Ausgang 48. Beispielsweise werden die Flusspumpe-Eingang 46 und die Flusspumpe-Ausgang 48 mit einer Flussratendifferenz von etwa 400 ml/min betrieben, derart, dass mehr (Dialysier-) Flüssigkeit von der Flusspumpe-Eingang 46 in den Dialysator 6 gedrückt wird als von der Flusspumpe-Ausgang 48 entzogen wird.

Durch diesen großen Fluss(raten)unterschied auf der Dialysierflüssigkeitsseite wird ein relativ großer (betragsmäßig) negativer Transmembrandruck (TMP) in dem Dialysator 6 erzeugt. Offenbarungsgemäß wird, damit der Transmembrandruck (TMP) nicht den maximal zulässigen Transmembrandruck unterschreitet bzw. betragsmäßig überschreitet, der Kompressor bzw. die Pegel-/ Levelregulierungspumpe 32 in dem extrakorporalen Kreislauf 4 geeignet angesteuert. Der Kompressor 32 kann grundsätzlich sowohl Luft in den extrakorporalen Kreislauf/ das A/V-Schlauchsystem 4 drücken, als auch Luft aus dem extrakorporalen Kreislauf/ dem A/V-Schlauchsystem 4 entfernen.

Der Kompressor 32 unterstützt im Zug-/ bzw. Pull-Zyklus B durch ein Herausziehen der Luft aus dem extrakorporalen Kreislauf 4 den Übertritt der (Dialysier-)Flüssigkeit durch die Membran 10 des Dialysators 6. Da beim Herausziehen bzw. Entfernen von Luft aus dem extrakorporalen Kreislauf die vorgesehene Luftfalle bzw. Expansionskammer 16 (erstes Ausführungsbeispiel) bzw. die vorgesehenen Luftfallen bzw. Expansionskammern 16 und 56 (zweites Ausführungsbeispiel) kollabieren kann/ können, wird gemäß der vorliegenden Offenbarung ein weiterer Zyklus, nämlich der Druck-/ bzw. Push-Zyklus A in den Verfahrens-/ bzw. Steuerungsablauf integriert. In dem Druck-/ bzw. Push-Zyklus wird Luft durch den Kompressor/ die Pegel-/ Levelregulierungspumpe 32 in den extrakorporalen Kreislauf 4 gedrückt. Der Druck-Zyklus neutralisiert dabei den in dem extrakorporalen Kreislauf 4 vorherrschenden Unterdruck stückweise. Ein Wechsel der Zyklen A und B kann grundsätzlich sowohl sensorgesteuert als auch zeitgesteuert vorgenommen werden.

Zu Beginn des Verfahrens-/ bzw. Steuerungsablaufs wird bevorzugt der Druck-Zyklus A ausgeführt, welcher den Transmembrandruck positiv beeinflusst. In anderen Worten steigt der Transmembrandruck, wenn Luft von dem Kompressor 32 in den extrakorporalen Kreislauf 4 gedrückt wird. Wenn der Transmembrandruck einen ersten vorbestimmten Schwellwert x überschreitet, welcher beispielsweise bei -400 mmHg liegt, erfolgt ein Wechsel in den Zug-Zyklus B. In dem Zug-Zyklus B wird so lange Luft über den Kompressor 32 aus dem extrakorporalen Kreislauf 4 gezogen, bis der Transmembrandruck einen zweiten vorbestimmten Schwellwert y unterschreitet, welcher beispielsweise bei -420 mmHg liegt. Dann wird wieder der bereits zuvor beschriebene Druck-Zyklus A ausgeführt.

Der Druck-Zyklus A und der Zug-Zyklus B werden daher dem Schaubild der Fig. 4 entsprechend zyklisch wiederholt.

In Fig. 5 ist der Verlauf des Transmembrandrucks bei Ausführung des Push-Pull-Verfahrens gemäß der vorliegenden Offenbarung veranschaulicht. Hier wird deutlich, dass auch wenn von dem Zug-Zyklus B zu dem Druck-Zyklus A bei y gewechselt wird, der Transmembrandruck zunächst weiter abfällt, bevor dieser nach Erreichen eines unteren Scheitelpunkts schließlich in dem Druck-Zyklus positiv beeinflusst wird und entsprechend ansteigt. Auch wenn dann wiederum von dem Druck-Zyklus A zu dem Zug-Zyklus B bei x gewechselt wird, fällt auf, dass der Transmembrandruck zunächst weiter ansteigt, bevor dieser nach Erreichen eines oberen Scheitelpunkts schließlich in dem Zug-Zyklus negativ beeinflusst wird und entsprechend abfällt.

Wenn der erste vorbestimmte Schwellwert x und der zweite vorbestimmte Schwellwert y geeignet gewählt werden, kann erreicht werden, dass sich der Transmembrandruck TMP in einem gewünschten Bereich bewegt und einen maximal zulässigen Transmembrandruck nicht unterschreitet bzw. betragsmäßig überschreitet. Wie aus den Zeitangaben in Fig. 5 hervorgeht, erfolgt der Wechsel zwischen Druck-Zyklus A und Zug-Zyklus B in relativ kurzen zeitlichen Abständen von etwa 0,5 Sekunden.

Offenbarungemäß kann es vorgesehen sein, dass beim Push-Pull-Verfahren die venöse Schlauchklemme 20 in geeigneter Weise angesteuert (das heißt geöffnet oder geschlossen) wird, um das Push-Pull-Verfahren zu unterstützen. Beispielsweise kann im Zug-Zyklus ein Unterdruck schneller aufgebaut/ erreicht werden, wenn die venöse Schlauchklemme 20 geschlossen ist. Weiterhin besteht, wenn High-Flux-Dialysatoren für das offenbarungsgemäße Primen verwendet werden, die Gefahr dass sich die venöse Luftfalle 16 (erstes Ausführungsbeispiel) bzw. die venöse Luftfalle 16 und die arterielle Luftfalle 56 (zweites Ausführungsbeispiel) mit (Dialysier-) Flüssigkeit füllen und gegebenenfalls die Hydrophobfilter 40, 58 benetzt werden, so dass es bei High-Flux-Dialysatoren erforderlich sein kann, in vorbestimmten kurzen Zeitintervallen (beispielsweise alle 0,5 Sekunden) die venöse Schlauchklemme 20 kurz zu öffnen.

Das offenbarungsgemäße Push-Pull-Verfahren stellt im Wesentlichen eine Kompromisslösung bereit, welche sowohl für High-Flux-Dialysatoren als auch für Low-Flux-Dialysatoren anwendbar ist. Es hat sich gemäß der vorliegenden Offenbarung jedoch als vorteilhaft herausgestellt, wenn zumindest zwei verschiedene Push-Pull-Verfahren, nämlich das "Push/Pull universal"-Verfahren (siehe (4) in Fig. 3) und ein "Push/Pull Low Flux"-Verfahren (siehe (5) in Fig. 3), ausgeführt werden.

Beispielsweise kann von (4) "Push/Pull universal" zu (5) "Push/Pull Low Flux" geschaltet werden, wenn eine vorbestimmte Zeitdauer, für welche "Push/Pull universal" durchgeführt wird, überschritten wird. Diese vorbestimmte Zeitdauer kann beispielsweise bei etwa 75 Sekunden angesetzt werden.

Insbesondere kann das "Push/Pull universal"-Verfahren, welches gemäß der vorliegenden Offenbarung zuerst ausgeführt wird, eher an die Anforderungen bei High-Flux-Dialysatoren angepasst sein (beispielsweise kann der Zug-Zyklus kürzer eingestellt werden), während hingegen das "Push/Pull Low Flux"-Verfahren speziell an die Anforderungen bei Low-Flux-Dialysatoren angepasst sein kann (beispielsweise kann der Zug-Zyklus länger eingestellt werden oder die venöse Schlauchklemme 20 durchgehend geschlossen sein).

Wenn beim "Push/Pull universal"-Verfahren bereits eine längere Zeitdauer verstrichen ist und noch kein Abbruch des Verfahrens erfolgt ist, deutet dies darauf hin, dass sich der Übertritt von (Dialysier-)Flüssigkeit über die Membran 10 des Dialysators 6 schwierig gestaltet, und es sich bei dem verwendeten Dialysator 6 um einen Low-Flux-Dialysator handelt. In diesem Fall ist offenbarungsgemäß vorgesehen, ein an einen Low-Flux-Dialysator angepasstes Push-Pull-Verfahren auszuführen.

Das Push-Pull-Verfahren wird letztendlich abgebrochen, sobald der Sicherheitsluftdetektor 18 Flüssigkeit (Dialysierflüssigkeit) erfasst. Sobald dies der Fall ist, erfolgt ein Übergang zu (6) "Priming füllen langsam" (von (4) oder von (5)) in Fig. 3.

Bei Schritt (6) wird der extrakorporale Kreislauf 4 langsam mit (Dialysier-)Flüssigkeit gefüllt. Dabei werden die Flusspumpe-Eingang 46 und die Flusspumpe-Ausgang 48 weiterhin bevorzugt mit einer Flussratendifferenz betrieben. Beispielsweise kann eine Flussratendifferenz von 400 ml/min vorgesehen sein, wobei für die Flusspumpe-Eingang 46 eine Flussrate von 600 ml/min und für die Flusspumpe-Ausgang 48 eine Flussrate von 200 ml/min eingestellt wird. Die arterielle Blutpumpe 24 wird dabei bevorzugt in Vorwärtsrichtung, beispielsweise bei 150 ml/min betrieben. Die arterielle Schlauchklemme 22 und die venöse Schlauchklemme 20 sind bevorzugt geöffnet.

Grundsätzlich gilt, dass auch bei Schritt (6) der Kompressor bzw. die Pegel-/ bzw. Levelregulierungspumpe 32 in geeigneter Weise angesteuert werden kann (beispielsweise durch Herausziehen von Luft aus dem extrakorporalen Kreislauf 4), um den Schritt "Priming füllen langsam" zu unterstützen. Beispielsweise kann, wenn der Sicherheitsluftdetektor 18 keine (Dialysier-)Flüssigkeit (mehr) erfasst, also der Flüssigkeitsfluss nicht kontinuierlich ist, und gleichzeitig der Transmembrandruck über einem Schwellwert (beispielsweise -420 mmHg) liegt, der Kompressor 32 Luft aus dem extrakorporalen Kreislauf 4 herausziehen. Der Kompressor 32 kann wieder angehalten werden, wenn der Transmembrandruck unter dem Schwellwert ist, oder wenn der venöse Drucksensor 30 einen Druck misst, welcher unter einem vorbestimmten Schwellwert (beispielsweise 200 mmHg) liegt.

Bei Schritt (6) "Priming füllen langsam" steigt der Druck in dem extrakorporalen Kreislauf 4 grundsätzlich kontinuierlich an. Wenn der venöse Drucksensor 30 einen Druck feststellt, welcher über einem vorbestimmten Schwellwert, beispielsweise 500 mmHg, liegt, ist der extrakorporale Kreislauf 4 weitestgehend gefüllt und das Verfahren geht weiter zu Schritt (7) "Priming füllen schnell".

Der Schritt (7) "Priming füllen schnell" ist grundsätzlich dem Schritt (6) "Priming füllen langsam" sehr ähnlich. Es werden lediglich die Förder- bzw. Flussraten der arteriellen Blutpumpe 24, der Flusspumpe-Eingang 46 und der Flusspumpe-Ausgang 48 verändert.

Bevorzugt wird dabei die Flussrate der arteriellen Blutpumpe 24 erhöht, beispielsweise von 150 ml/min auf 300 ml/min. Dadurch werden noch vorhandene Luftblasen mitgerissen und an der venösen Luftfalle 16 (erstes Ausführungsbeispiel) bzw. der venösen Luftfalle 16 und der arteriellen Luftfalle 56 (zweites Ausführungsbeispiel) extrahiert. Die Flussratendifferenz an der Dialysierflüssigkeitsseite wird bevorzugt angehoben, beispielsweise von 400 ml/min auf 600 ml/min, bei einer Flussrate der Flusspumpe-Eingang 46 von 700 ml/min und einer Flussrate der Flusspumpe-Ausgang 48 von 100 ml/min. Bei einer erhöhten Flussrate auf der Seite des extrakorporalen Kreislaufs 4 kann grundsätzlich ein schlechteres Übertreten von (Dialysier-)Flüssigkeit über die Membran 10 des Dialysators 6 beobachtet werden. Daher wurde auf der Seite des Dialysierflüssigkeitskreislaufs 8 die Flussratendifferenz erhöht, um das Übertreten zu verbessern.

Damit die (Dialysier-)Flüssigkeit im extrakorporalen Kreislauf 4 zirkuliert, müssen die venöse Schlauchklemme 20 und die arterielle Schlauchklemme 22 geöffnet sein. Analog zu dem vorherigen Schritt (6) "Priming füllen langsam" kann der Kompressor bzw. die Levelregulierungspumpe 32 bei einem diskontinuierlichen Flüssigkeitsfluss im extrakorporalen Kreislauf 4 in geeigneter Weise angesteuert werden, um den Flüssigkeitspegel in der Luftfalle 16 bzw. den Luftfallen 16, 56 anzuheben. Damit sich die Luftfalle 16 bzw. die Luftfallen 16, 56 bei aktiviertem Kompressor 32 nicht unkontrolliert füllen, wird dieser ausgeschaltet, wenn der venöse Drucksensor einen Druck feststellt, welcher kleiner einem vorbestimmten Wert, beispielsweise 200 mmHg, ist. Schritt (7) wird zeitgesteuert abgebrochen, insbesondere wenn eine vorbestimmte Zeitdauer verstrichen ist, beispielsweise 25 Sekunden.

Nach dem zeitgesteuerten Abbruch von Schritt (7) "Priming füllen schnell" geht der Ablauf optional weiter zu Schritt (8) "Senken venöser Druck". Schritt (8) kann grundsätzlich bei allen Low-Flux-Dialysatoren und den meisten High-Flux-Dialysatoren angewendet werden. Ziel des Schritts (7) ist es, den venösen Druck, welcher in den beiden vorherigen Schritten (5) und (6) stark angestiegen ist, wieder zu senken. Um den venösen Druck zu senken, werden bevorzugt die Ventile vor dem Kompressor bzw. der Levelregulierungspumpe 32, also das erste Ventil 34, das zweite Ventil 36 und das dritte Ventil 38, so geschaltet, dass ein Druckausgleich mit der Umgebungsluft erfolgen kann. Dadurch steigt grundsätzlich der Pegel in der venösen Luftfalle 16. Um ein unkontrolliertes Steigen des Pegels der venösen Luftfalle 16 zu verhindern, wird Schritt (7) bevorzugt abgebrochen, wenn ein vorbestimmter Schwellwert des venösen Drucks, beispielsweise 200 mmHg, unterschritten wird.

Bei Schritt (8) werden die Flusspumpe-Eingang 46 und die Flusspumpe-Ausgang 48 mit einer geringen Flussratendifferenz betrieben. Beispielsweise wird die Flussrate der Flusspumpe-Eingang 46 auf 500 ml/min, und die Flussrate der Flusspumpe-Ausgang 48 auf 470 ml/min eingestellt. Die arterielle Blutpumpe 24 dreht sich bevorzugt rechtsdrehend, das heißt in Vorwärtsrichtung, beispielsweise bei einer Flussrate von 150 ml/min. Die venöse Schlauchklemme 20 und die arterielle Schlauchklemme 22 sind bevorzugt geöffnet, damit die Flüssigkeit in dem extrakorporalen Kreislauf 4 zirkulieren kann.

Nach Schritt (8) geht der Verfahrensablauf weiter zu Schritt (9) "Zirkulieren rückwärts". In diesem Schritt wird die arterielle Blutpumpe 24 in Rückwärtsrichtung, das heißt linksdrehend, beispielsweise bei einer Flussrate von 300 ml/min betrieben, um noch vorhandene Luftblasen aus dem Dialysator 6 zu entfernen. Auch bei Schritt (9) werden die Flusspumpe-Eingang 46 und die Flusspumpe-Ausgang 48 mit einer geringen Flussratendifferenz betrieben (beispielsweise Flusspumpe-Eingang 46 bei 500 ml/min und Flusspumpe-Ausgang 48 bei 470 ml/min). Die venöse Schlauchklemme 20 und die arterielle Schlauchklemme 22 sind bevorzugt weiterhin geöffnet. Der Kompressor 32 wird bevorzugt nicht angesteuert. Bevorzugt wird dieser Schritt lediglich bei der extrakorporalen Blutbehandlungsvorrichtung 2 gemäß der in Fig. 1 gezeigten ersten Ausführungsform ausgeführt. Schritt (9) wird bevorzugt zeitgesteuert (beispielsweise nach etwa 25 Sekunden) abgebrochen.

Nach Schritt (9) geht der Verfahrensablauf weiter zu Schritt (10) "Zirkulieren vorwärts". In diesem Verfahrensschritt wird die Flüssigkeit in Vorwärtsrichtung (rechtsdrehend) zirkuliert, um noch vorhandene Luftblasen in dem extrakorporalen Kreislauf 4, die während des Rückwärtszirkulierens aus dem Dialysator 6 extrahiert wurden, über die venöse Luftfalle 16 abzuscheiden. Grundsätzlich werden die Flusspumpe-Eingang 46 und die Flusspumpe-Ausgang 48 mit geringer Flussratendifferenz (Therapieparameter), beispielsweise die Flusspumpe-Eingang 46 bei 500 ml/min und die Flusspumpe-Ausgang 48 bei 470 ml/min betrieben, die arterielle Blutpumpe 24 dreht in Vorwärtsrichtung (rechtsdrehend), etwa bei 300 ml/min, und die venöse Schlauchklemme 20 und die arterielle Schlauchklemme 22 sind geöffnet.

Wie bei Schritt (6) "Priming füllen langsam" kann jedoch der Kompressor 32 grundsätzlich auch Luft aus dem extrakorporalen Kreislauf 4 ziehen, insbesondere wenn der Sicherheitsluftdetektor 18 keine (Dialysier-)Flüssigkeit mehr registriert und der Transmembrandruck über einem vorbestimmten Schwellwert, beispielsweise -420 mmHg liegt. In diesem Fall kann ein größerer Flussunterschied (z.B. 600 ml/min) zwischen der Flusspumpe-Eingang 46 und der Flusspumpe-Ausgang 48 eingestellt werden. Beispielsweise könnte die Flussrate der Flusspumpe-Eingang 46 auf 700 ml/min und die Flussrate der Flusspumpe-Ausgang 48 auf 100 ml/min eingestellt werden. Durch die größere Flussdifferenz kann erreicht werden, dass mehr (Dialysier- )Flüssigkeit durch die Membran 10 des Dialysators 6 tritt und sich somit ein kontinuierlicher Flüssigkeitsfluss in dem extrakorporalen Kreislauf 4, insbesondere in dem venösen Abschnitt 14 hinter der venösen Luftfalle 16 einstellt.

Bevorzugt wird hier der Kompressor 32 angehalten (Abbruchkriterium), wenn der venösen Drucksensor 30 einen venösen Druck misst, welcher kleiner 0 mmHg ist. Das Abbruchkriterium für das Ziehen des Kompressors 32 hat sich somit im Vergleich zu den vorhergehenden Schritten geändert. Grund dafür ist, dass der venöse Druck während der Schritte (9) und (10) bereits (monoton) gesunken ist, während kontinuierlich Luft aus dem extrakorporalen Kreislauf 4 extrahiert wurde. Aus diesem Grund ist es erforderlich, dass auch eine Sicherheitsschwelle der Pegelregulierung sinkt.

Wie zuvor beschrieben wurde, zieht der Kompressor bzw. die Levelregulierungspumpe 32 Luft aus dem extrakorporalen Kreislauf 4, sobald der Sicherheitsluftdetektor 18 Luft registriert. Dabei steigt der Pegel der venösen Luftfalle 16. Durch das vorherige Rückwärtszirkulieren sind viele Luftblasen in dem extrakorporalen Kreislauf 4 vorhanden. Falls an dem Sicherheitsluftdetektor 18 eine Reihe von Luftblasen existieren, würde das zu einem unkontrollierten Steigen des Pegels in der venösen Luftfalle 16 führen, da das Ziehen durch den Kompressor 32 mehrfach hintereinander ausgeführt werden würde. Um diesem ungewollten Ablauf entgegen zu wirken, ist eine Sperrung des Ziehens in einem vorbestimmten Zeitraum (beispielsweise den ersten 5 Sekunden) erforderlich. Danach kann ein normaler Programmablauf wieder aufgenommen werden.

Schritt (10) "Zirkulieren vorwärts" wird grundsätzlich beendet, wenn eine vorbestimmte Zeit, beispielsweise 25 Sekunden, verstrichen ist.

Nach Schritt (10) wird bei Schritt (11) "Luftfalle stellen" der Pegel der venösen Luftfalle 16 (für das erste Ausführungsbeispiel) bzw. die Pegel der venösen Luftfalle 16 und der arteriellen Luftfalle 56 (für das zweite Ausführungsbeispiel) eingestellt.

Wenn die Vorbereitung der extrakorporalen Blutbehandlungsvorrichtung 2 insgesamt abgeschlossen ist, kann bei Schritt (12) eine Therapie erfolgen. Dabei wird die Verbindung zwischen dem arteriellen Abschnitt 12 und dem venösen Abschnitt 14 (an dem Adapter/ Verbinder 15) gelöst und der arterielle Abschnitt 12 wird an einen Patienten angeschlossen. Die arterielle Blutpumpe 24 rotiert und saugt das Blut aus dem Patienten. In dem extrakorporalen Kreislauf 4 vorhandene (Dialysier-)Flüssigkeit wird abgelassen. Sobald der extrakorporale Kreislauf 4 ausreichend mit Blut gefüllt ist, wird auch der venöse Abschnitt 14 an den Patienten angeschlossen und die Therapie kann beginnen.

Die Gesamtlaufzeit des in Fig. 3 dargestellten Prozesses beträgt bei High-Flux-Dialysatoren etwa 4 bis 5 min, und bei Low-Flux-Dialysatoren etwa 10 bis 15 min.

### Bezugszeichenliste

- 2: extrakorporale Blutbehandlungsvorrichtung
- 4: extrakorporaler Kreislauf
- 6: Dialysator
- 8: Dialysierflüssigkeitskreislauf
- 10: Membran
- 12: arterieller Abschnitt
- 14: venöser Abschnitt
- 15: Adapter/ Verbinder
- 16: venöse Expansionskammer/ Luftfalle
- 18: venöser Sicherheitsluftdetektor
- 20: venöse Schlauchklemme
- 22: arterielle Schlauchklemme
- 24: arterielle Blutpumpe
- 26: arterieller Drucksensor
- 28: Dialysatoreingangsdrucksensor
- 30: venöser Drucksensor
- 32: Kompressor/ Pegel-/ Levelregulierungspumpe
- 34: erstes Ventil
- 36: zweites Ventil
- 38: drittes Ventil
- 40: Hydrophobfilter
- 42: Dialysatoreinlassventil
- 44: Dialysatorauslassventil
- 46: Flusspumpe-Eingang
- 48: Flusspumpe-Ausgang
- 50: Dialysierflüssigkeitszufluss
- 52: Dialysierflüssigkeitsabfluss
- 54: Steuereinheit
- 56: arterielle Expansionskammer/ Luftfalle
- 58: Hydrophobfilter
- A: Push-/ bzw. Druck-Zyklus
- B: Pull-/ bzw. Zug-Zyklus
- x: erster vorbestimmter Schwellwert
- y: zweiter vorbestimmter Schwellwert

## Patentansprüche

1. Extrakorporale Blutbehandlungsvorrichtung (2), welche zum automatischen Primen derselben vorbereitet ist, mit:
einem extrakorporalen Kreislauf (4), in welchem eine Strömungsmaschine (32), insbesondere Kompressor oder Pumpe, vorgesehen ist, einem Dialysator (6), einem Dialysierflüssigkeitskreislauf (8) und einer Steuereinheit (54), wobei
der extrakorporale Kreislauf (4) und der Dialysierflüssigkeitskreislauf (8) über eine in dem Dialysator (6) vorgesehene Membran (10) voneinander getrennt sind,
die Steuereinheit (54) eingerichtet ist,
das Primen derart zu steuern, dass eine Flüssigkeit, insbesondere Priming-/ Dialysierflüssigkeit, von dem Dialysierflüssigkeitskreislauf (8) über die Membran (10) des Dialysators (6) an den extrakorporalen Kreislauf (4) geliefert wird, und
in dem Dialysierflüssigkeitskreislauf (8) vorgesehene Ventile (42, 44) und/oder Pumpen (46, 48) derart anzusteuern, dass ein Druckaufbau in dem Dialysator (6) auf der Dialysierflüssigkeitsseite erfolgt,
**dadurch gekennzeichnet, dass**
die Steuereinheit (54) eingerichtet ist, die in dem extrakorporalen Kreislauf (4) vorgesehene Strömungsmaschine (32) anzusteuern, insbesondere zyklisch alternierend, einen Push-/ bzw. Druck-Zyklus (A), in welchem die Strömungsmaschine (32) Luft in den extrakorporalen Kreislauf (4) drückt, und einen Pull-/ bzw. Zug-Zyklus (B), in welchem die Strömungsmaschine (32) Luft aus dem extrakorporalen Kreislauf (4) herauszieht, auszuführen, zum Unterstützen eines Übertritts der Flüssigkeit über die Membran (10) des Dialysators (6) beim Primen.

2. Extrakorporale Blutbehandlungsvorrichtung (2) nach Anspruch 1, **dadurch gekennzeichnet, dass** in dem Dialysierflüssigkeitskreislauf (8) stromaufwärts des Dialysators (6) eine Flusspumpe-Eingang (46) und stromabwärts des Dialysators (6) eine Flusspumpe-Ausgang (48) vorgesehen ist, und die Steuereinheit (54) eingerichtet ist, zum Druckaufbau in dem Dialysator (6) auf der Dialysierflüssigkeitsseite die Flusspumpe-Eingang (46) und die Flusspumpe-Ausgang (48) derart anzusteuern, dass eine Flussrate der Flusspumpe-Eingang (46) größer ist als eine Flussrate der Flusspumpe-Ausgang (48).

3. Extrakorporale Blutbehandlungsvorrichtung (2) nach Anspruch 1 oder 2,
**dadurch gekennzeichnet, dass** die in dem extrakorporalen Kreislauf (4) vorgesehene Strömungsmaschine (32) ein Kompressor bzw. eine Pegel-/ Levelregulierungspumpe ist, welcher bzw. welche hinter einer in dem extrakorporalen Kreislauf (4) vorgesehenen Expansionskammer (16, 56) angeordnet ist bzw. mit der Expansionskammer (16, 56) in Verbindung steht, und die Luft in die Expansionskammer (16, 56) drückt oder aus dieser herauszieht.

4. Extrakorporale Blutbehandlungsvorrichtung (2) nach einem der vorhergehenden Ansprüche 1 bis 3, **dadurch gekennzeichnet, dass** die Steuereinheit (54) eingerichtet ist, den Push-/ bzw. Druck-Zyklus (A) und den Pull-/ bzw. Zug-Zyklus (B) zyklisch abzuwechseln und zu wiederholen.

5. Extrakorporale Blutbehandlungsvorrichtung (2) nach Anspruch 4, **dadurch gekennzeichnet, dass** die Steuereinheit (54) eingerichtet ist, das zyklische Alternieren des Push-/ bzw. Druck-Zyklus (A) und des Pull-/ bzw. Zug-Zyklus (B) abzubrechen, wenn ein in einem venösen Abschnitt (14) des extrakorporalen Kreislaufs (4) vorgesehener Sicherheitsluftdetektor (18) die Flüssigkeit, insbesondere Dialysierflüssigkeit, erfasst.

6. Extrakorporale Blutbehandlungsvorrichtung (2) nach einem der vorhergehenden Ansprüche 1 bis 5, **dadurch gekennzeichnet, dass** der Dialysator (6) ein Low-Flux-Dialysator mit einem Ultrafiltrationskoeffizienten von kleiner 15 ml/(h*mmHg) ist.

7. Extrakorporale Blutbehandlungsvorrichtung (2) nach einem der vorhergehenden Ansprüche, **dadurch gekennzeichnet, dass** die Steuereinheit (54) eingerichtet ist, einen Wechsel zwischen dem Push-/ bzw. Druck-Zyklus (A) und dem Pull-/ bzw. Zug-Zyklus (B) sensorgesteuert und/oder zeitgesteuert vorzunehmen.

8. Extrakorporale Blutbehandlungsvorrichtung (2) nach einem der vorhergehenden Ansprüche 1 bis 7, **dadurch gekennzeichnet, dass** die Steuereinheit (54) eingerichtet ist, zuerst den Push-/ bzw. Druck-Zyklus (A) durchzuführen und nach dem erstmaligen Durchführen des Push-/ bzw. Druck-Zyklus (A) den Pull-/ bzw. Zug-Zyklus (B) durchzuführen.

9. Extrakorporale Blutbehandlungsvorrichtung (2) nach einem der vorhergehenden Ansprüche 1 bis 8, **dadurch gekennzeichnet, dass** die Steuereinheit (54) eingerichtet ist, von dem Push-/ bzw. Druck-Zyklus (A) zu dem Pull-/ bzw. Zug-Zyklus (B) zu wechseln, wenn ein Transmembrandruck (TMP) einen ersten vorbestimmten Schwellwert (x) überschreitet, und von dem Pull-/ bzw. Zug-Zyklus (B) zu dem Push-/ bzw. Druck-Zyklus (A) zu wechseln, wenn der Transmembrandruck einen zweiten vorbestimmten Schwellwert (y) unterschreitet.

10. Extrakorporale Blutbehandlungsvorrichtung nach Anspruch 9, **dadurch gekennzeichnet, dass** der erste vorbestimmte Schwellwert (x) größer als der zweite vorbestimmte Schwellwert (y) ist.

11. Verfahren zum automatischen Primen einer extrakorporalen Blutbehandlungsvorrichtung (2) umfassend einen extrakorporalen Kreislauf (4), einen Dialysator (6) und einen Dialysierflüssigkeitskreislauf (8) mit den Schritten:
Primen des extrakorporalen Kreislaufs (4) und des Dialysators (6), indem eine Flüssigkeit von dem Dialysierflüssigkeitskreislauf (8) über eine Membran (10) des Dialysators (6) an den extrakorporalen Kreislauf (4) geliefert wird;
Aufbauen eines Drucks in dem Dialysator (6) auf der Dialysierflüssigkeitsseite durch geeignetes Ansteuern bzw. Betätigen von in dem Dialysierflüssigkeitskreislauf (8) vorgesehenen Ventilen (42, 44) und/oder Pumpen (46, 48); und
Zyklisches Alternieren eines Push-/ bzw. Druck-Zyklus (A), in welchem eine in dem extrakorporalen Kreislauf (4) vorgesehene Strömungsmaschine (32), insbesondere Kompressor oder Pumpe, Luft in den extrakorporalen Kreislauf (4) drückt, und eines Pull-/ bzw. Zug-Zyklus (B), in welchem die Strömungsmaschine (32) Luft aus dem extrakorporalen Kreislauf (4) herauszieht, zum Unterstützen eines Übertritts der Flüssigkeit über die Membran (10) des Dialysators (6) beim Primen.

## Claims

1. An extracorporeal blood treatment device (2) prepared for automatic priming thereof, comprising:
an extracorporeal circuit (4), in which a flow machine (32), in particular a compressor or pump, is provided, a dialyzer (6), a dialysis liquid circuit (8) and a control unit (54), wherein
the extracorporeal circuit (4) and the dialysis liquid circuit (8) are separated from each other via a membrane (10) provided in the dialyzer (6),
wherein the control unit (54) is configured
to control the priming in such a way that a liquid, in particular priming/dialysis liquid, is supplied from the dialysis liquid circuit (8) via the membrane (10) of the dialyzer (6) to the extracorporeal circuit (4), and
to control valves (42, 44) and/or pumps (46, 48) provided in the dialysis liquid circuit (8) in such a way that a pressure build-up takes place in the dialyzer (6) on the dialysis liquid side,
**characterized in that**
the control unit (54) is configured to control the flow machine (32) provided in the extracorporeal circuit (4), in particular cyclically alternating, to perform a push/ or press cycle (A), in which the flow machine (32) pushes air into the extracorporeal circuit (4), and a pull/ or suck cycle (B), in which the flow machine (32) pulls air out of the extracorporeal circuit (4), for supporting a transfer of the liquid via the membrane (10) of the dialyzer (6) during priming.

2. The extracorporeal blood treatment device (2) according to claim 1,
**characterized in that** a flux-pump inlet (46) is provided in the dialysis liquid circuit (8) upstream of the dialyzer (6) and a flux-pump outlet (48) is provided downstream of the dialyzer (6), and the control unit (54) is configured to control the flux-pump inlet (46) and the flux-pump outlet (48) to build up pressure in the dialyzer (6) on the dialysis liquid side in such a way that a flow rate of the flux-pump inlet (46) is greater than a flow rate of the flux-pump outlet (48).

3. The extracorporeal blood treatment device (2) according to claim 1 or 2,
**characterized in that** the flow machine (32) provided in the extracorporeal circuit (4) is a compressor or a gauge/level regulation pump, respectively, which is arranged downstream of an expansion chamber (16, 56) provided in the extracorporeal circuit (4) or which is connected to the expansion chamber, and which presses the air into expansion chamber (16, 56) or sucks it out of the expansion chamber (16, 56).

4. The extracorporeal blood treatment device (2) according to one of the preceding claims 1 to 3, **characterized in that** the control unit (54) is configured to cyclically alternate and repeat the push/ or press cycle (A) and the pull/ or suck cycle (B).

5. The extracorporeal blood treatment device (2) according to claim 4,
**characterized in that** the control unit (54) is configured to stop the cyclic alternation of the push/ or press cycle (A) and the pull/ or suck cycle (B) when a safety air detector (18) provided in a venous portion (14) of the extracorporeal circuit (4) detects the liquid, in particular dialysis liquid.

6. The extracorporeal blood treatment device (2) according to one of the preceding claims 1 to 5, **characterized in that** the dialyzer (6) is a low-flux dialyzer with an ultrafiltration coefficient of less than 15 ml/(h*mmHg).

7. The extracorporeal blood treatment device (2) according to one of the preceding claims, **characterized in that** the control unit (54) is configured to switch between the push/ or press cycle (A) and the pull/ or suck cycle (B) in a sensor-controlled and/or time-controlled manner.

8. The extracorporeal blood treatment device (2) according to one of the preceding claims 1 to 7, **characterized in that** the control unit (54) is configured to first perform the push/ or press cycle (A) and to perform the pull/ or suck cycle (B) after performing the push/ or press cycle (A) for the first time.

9. The extracorporeal blood treatment device (2) according to one of the preceding claims 1 to 8, **characterized in that** the control unit (54) is configured to switch from the push/ or press cycle (A) to the pull/ or suck cycle (B) when a transmembrane pressure (TMP) exceeds a first predetermined threshold value (x), and to switch from the pull/ or suck cycle (B) to the push/ or press cycle (A) when the transmembrane pressure falls below a second predetermined threshold value (y).

10. The extracorporeal blood treatment device of claim 9, **characterized in that** the first predetermined threshold value (x) is greater than the second predetermined threshold value (y).

11. A method for automatically priming an extracorporeal blood treatment device (2) comprising an extracorporeal circuit (4), a dialyzer (6) and a dialysis liquid circuit (8), comprising the steps of:
priming the extracorporeal circuit (4) and the dialyzer (6) by supplying a liquid from the dialysis liquid circuit (8) to the extracorporeal circuit (4) via a membrane (10) of the dialyzer (6);
building up pressure in the dialyzer (6) on the dialysis liquid side by suitably controlling or actuating valves (42, 44) and/or pumps (46, 48) provided in the dialysis liquid circuit (8); and
cyclically alternating a push/ or press cycle (A), in which a flow machine (32), in particular a compressor or pump, provided in the extracorporeal circuit (4) pushes air into the extracorporeal circuit (4), and a pull/ or suck cycle (B), in which the flow machine (32) pulls air out of the extracorporeal circuit (4), for supporting transfer of the liquid via the membrane (10) of the dialyzer (6) during priming.

## Revendications

1. Dispositif de traitement sanguin extracorporel (2) qui est préparé pour l'amorçage automatique de celui-ci, avec :
un circuit extracorporel (4), dans lequel une machine d'écoulement (32), en particulier un compresseur ou une pompe, est prévue, un dialyseur (6), un circuit de liquide de dialyse (8) et un dispositif de commande (54), dans lequel
le circuit extracorporel (4) et le circuit de liquide de dialyse (8) sont séparés l'un de l'autre par le biais d'une membrane (10) prévue dans le dialyseur (6),
le dispositif de commande (54) est conçu
afin de commander l'amorçage de telle manière qu'un liquide, en particulier liquide d'amorçage/de dialyse, soit fourni par le circuit de liquide de dialyse (8) par le biais de la membrane (10) du dialyseur (6) au circuit extracorporel (4), et
de commander des vannes (42, 44) et/ou des pompes (46, 48) prévues dans le circuit de liquide de dialyse (8) de telle manière qu'un établissement de pression dans le dialyseur (6) soit effectué sur le côté de liquide de dialyse,
**caractérisé en ce que**
le dispositif de commande (54) est conçu afin de commander la machine d'écoulement (32) prévue dans le circuit extracorporel (4), en particulier de réaliser par voie cyclique en alternance, un cycle de poussée (A), dans lequel la machine d'écoulement (32) pousse de l'air dans le circuit extracorporel (4), et un cycle de traction (B), dans lequel la machine d'écoulement (32) aspire l'air du circuit extracorporel (4) pour permettre un transfert du liquide par le biais de la membrane (10) du dialyseur (6) lors de l'amorçage.

2. Dispositif de traitement sanguin extracorporel (2) selon la revendication 1, **caractérisé en ce que** dans le circuit de liquide de dialyse (8) en amont du dialyseur (6), une entrée de pompe de flux (46) et en aval du dialyseur (6), une sortie de pompe de flux (48) sont prévues, et le dispositif de commande (54) est conçu afin de commander pour l'établissement de pression dans le dialyseur (6) sur le côté de liquide de dialyse l'entrée de pompe de flux (46) et la sortie de pompe de flux (48) de telle manière qu'un débit de l'entrée de pompe de flux (46) soit supérieur à un débit de la sortie de pompe de flux (48).

3. Dispositif de traitement sanguin extracorporel (2) selon la revendication 1 ou 2, **caractérisé en ce que** la machine d'écoulement (32) prévue dans le circuit extracorporel (4) est un compresseur ou une pompe de régulation de niveau qui est agencé(e) derrière une chambre de détente (16, 56) prévue dans le circuit extracorporel (4) ou est en liaison avec la chambre de détente (16, 56), et pousse l'air dans la chambre de détente (16, 56) ou l'en aspire.

4. Dispositif de traitement sanguin extracorporel (2) selon l'une quelconque des revendications précédentes 1 à 3, **caractérisé en ce que** le dispositif de commande (54) est conçu afin de changer et de répéter de manière cyclique le cycle de poussée (A) et le cycle de traction (B).

5. Dispositif de traitement sanguin extracorporel (2) selon la revendication 4, **caractérisé en ce que** le dispositif de commande (54) est conçu afin d'interrompre l'alternance cyclique du cycle de poussée (A) et du cycle de traction (B) lorsqu'un détecteur d'air de sécurité (18) prévu dans une section (14) veineuse du circuit extracorporel (4) détecte le liquide, en particulier du liquide de dialyse.

6. Dispositif de traitement sanguin extracorporel (2) selon l'une quelconque des revendications précédentes 1 à 5, **caractérisé en ce que** le dialyseur (6) est un dialyseur à flux faible avec un coefficient d'ultrafiltration inférieur à 15 ml/(h*mmHg).

7. Dispositif de traitement sanguin extracorporel (2) selon l'une quelconque des revendications précédentes, **caractérisé en ce que** le dispositif de commande (54) est conçu afin d'entreprendre de manière commandée par capteur et/ou commandée par le temps un changement entre le cycle de poussée (A) et le cycle de traction (B).

8. Dispositif de traitement sanguin extracorporel (2) selon l'une quelconque des revendications précédentes 1 à 7, **caractérisé en ce que** le dispositif de commande (54) est conçu afin de réaliser tout d'abord le cycle de poussée (A) et après la première réalisation du cycle de poussée (A) le cycle de traction (B).

9. Dispositif de traitement sanguin extracorporel (2) selon l'une quelconque des revendications précédentes 1 à 8, **caractérisé en ce que** le dispositif de commande (54) est conçu afin de passer du cycle de poussée (A) au cycle de traction (B) lorsqu'une pression de transmembrane (TMP) dépasse une première valeur seuil prédéterminée (x), et de passer du cycle de traction (B) au cycle de poussée (A) lorsque la pression de transmembrane reste en dessous d'une seconde valeur seuil (y) prédéterminée.

10. Dispositif de traitement sanguin extracorporel (2) selon la revendication 9, **caractérisé en ce que** la première valeur seuil prédéterminée (x) est supérieure à la seconde valeur seuil prédéterminée (y).

11. Procédé d'amorçage automatique d'un dispositif de traitement sanguin extracorporel (2) comprenant un circuit extracorporel (4), un dialyseur (6) et un circuit de liquide de dialyse (8) avec les étapes consistant à :
amorcer le circuit extracorporel (4) et le dialyseur (6) en ce qu'un liquide est fourni du circuit de liquide de dialyse (8) par le biais d'une membrane (10) du dialyseur (6) au circuit extracorporel (4) ;
établir une pression dans le dialyseur (6) sur le côté de liquide de dialyse par commande ou actionnement approprié de vannes (42, 44) et/ou de pompes (46, 48) prévues dans le circuit de liquide de dialyse (8) ; et
alterner de manière cyclique un cycle de poussée (A), dans lequel une machine d'écoulement (32) prévue dans le circuit extracorporel (4), en particulier compresseur ou pompe, pousse de l'air dans le circuit extracorporel (4), et un cycle de traction (B), dans lequel la machine d'écoulement (32) aspire de l'air du circuit extracorporel (4), pour permettre un transfert du liquide par le biais de la membrane (10) du dialyseur (6) lors de l'amorçage.
